# EUROPEAN PATENT APPLICATION

(11) **EP 4 624 585 A1**
(43) Date of publication of application: **01.10.2025**
(21) Application number: 25156721.0
(22) Date of filing: 10.02.2025
(51) Int. Cl.: C12P 7/6418

(54) **METHOD FOR OBTAINING AT LEAST ONE BIOPRODUCT FROM THE WASTE INTENDED FOR USE AS RAW MATERIALS**

(30) Priority: 26.03.2024 UA 202401559; 26.03.2024 UA 202401560
(71) Applicant: Vegoil Baltic, UAB, 12124 Vilnius (LT)
(72) Inventor: Leonidovich Boytsov, Mikhail, 81700 Lviv (UA)
(74) Representative: Metida

(57) **Abstract**

The invention is intended for the oil and fat industry, in particular for the process of processing oil and fat industry refining products - various types of waste, containing fats, oils, and their fractions, intended for use as raw materials - using enzymes.

A method for obtaining at least one bioproduct from waste intended for use as raw materials was developed, it provides for the following stages: the waste intended for use as a raw material, which contains at least one of the following substances: fats, oils or their fractions, is supplied; the raw material is mixed with water and heated to a temperature not lower than 40°C by mechanical stirring; enzyme is added; fermentation takes place under stirring and adjusting the temperature of the mixture, and at least one bioproduct is obtained; at least one bioproduct is separated. The liquid lipase product obtained from the genetically modified microorganism *Aspergillus oryzae* is used as an enzyme.

Thus, a method that allows obtaining at least one bioproduct from waste intended for use as raw materials was developed, which, after optimally choosing the conditions of its application, allows to ensure a technical result that would allow optimizing the temperature conditions during the implementation of the method steps, reducing the fermentation time, reducing energy costs, and obtaining a bioproduct with relatively low amount of harmful impurities.

## Description

The invention is intended for the oil and fat industry, in particular for the process of processing oil and fat industry refining products - various types of waste, containing fats, oils, and their fractions, intended for use as raw materials - using enzymes.

In nowadays, processes for the processing of by-products of the oil and fat industry using enzymes have quite favorable conditions for their conduction. The fermentation reaction has a relatively low working temperature, low energy consumption, and a rather high utilization rate of the waste intended for use as raw materials. In addition, this reaction meets the requirements for reducing harmful emissions and significantly reduces the impact of organic solvents and chemical reagents on human health and the environment. However, the process of searching for the most efficient, safest, and economically sound method to obtain bioproducts by fermentation of various types of waste intended for use as raw materials, containing fats, oils and their fractions, is still ongoing and has wide prospects.

Patent KHP No. 103436366 provides a neutral oil extraction method, when the raw material from which the oil is to be extracted is mixed with water or a buffer solution under the conditions of mechanical stirring or ultrasonic treatment, maintaining a solid-to-liquid mass ratio of 1:0.25 to 1:10, adding one or more phospholipases, and a fermentation reaction is induced at a temperature from 40°C to 60°C. At the end of the reaction, the resulting mixed solution is centrifuged to obtain the top layer of neutral oil.

The international application WO2013016690 describes a method for obtaining bioproducts from waste instended for use as raw materials, which includes the following steps: supplied raw materials containing fats, oils, and their fractions - by-products remaining after industrial processes such as biofuel production, fat saponification, production of alcoholic beverages, production of vegetable oils, or processes used in the oleochemical or petroleum refining industry. The raw materials are then heated to a temperature preferably between 45°C and 100°C, then mixed into the fermentation medium using at least one biocatalyst capable of fermenting with the available raw material. During this process, when the raw materials are mixed into the fermentation medium, a stable dispersion is formed. Also before placing the raw materials in the fermentation medium, lipases can be added to them at this stage. Later, the raw materials are mixed and fermented in a fermentation medium, producing at least one bioproduct. Hydrocarbons, triacylglycerols, or fatty acids can be separated in a coalescing filter system with the use of this method, this allows the two phases to be separated and then collected from the surface of the liquid. Using this invention, the following bioproducts can be obtained: organic acids (formic, vinegar, propionic, butter, citric, etc.), alcohols (methanol, ethanol, propanol, propanediol, etc.), hydroxy and dihydroxy acids, vitamins, enzymes, antibiotics, polyhydroxyalkanoate or polyhydroxybutyrate, but the list of substances is non-exaustive. Bioproducts also include (but are not limited to) ethanol, acetate, succinate, mevalonate, isoprene, and butyrate.

The shortcomings of these solutions - suboptimally chosen conditions for their implementation, such as the modes of the described methods and the substances used - led to a rather high heating temperature of the waste intended for use as raw materials, a rather high temperature during fermentation, as well as a rather long reaction time. For this reason, the implementation of the described technical solutions resulted in increased energy consumption and danger to personnel, and the implementation itself was more complicated.

Thus, the objective of the presented invention is to create a method of obtaining at least one bioproduct from waste intended for use as raw materials, which, after optimally choosing the conditions of its application, would ensure a technical result that would allow optimizing the temperature conditions during the implementation of the method steps, reducing the fermentation time, reducing energy costs, and obtaining a bioproduct with relatively low amount of harmful impurities.

The task was solved by creating the method of obtaining at least one bioproduct from waste intended for use as raw materials, which provides for the following stages: the waste intended for use as a raw material, which contains at least one of the following substances: fats, oils or their fractions, is supplied; the raw material is mixed with water and heated to a temperature not lower than 40°C by mechanical stirring; enzyme is added; fermentation takes place under stirring and adjusting the temperature of the mixture, and at least one bioproduct is obtained; at least one bioproduct is separated. The liquid lipase product obtained from the genetically modified microorganism *Aspergillus oryzae* is used as an enzyme.

It was experimentally established that the use of a liquid lipase product obtained from the genetically modified microorganism *Aspergillus oryzae* as an enzyme provides optimal conditions for the implementation of the presented method. In a preferred implementation of the present invention, the liquid lipase product is a hydrolase of carboxylic acid ester obtained by submerging the genetically modified microorganism *Aspergillus oryzae* in water for fermentation. To sum up, the aforementioned experimentally selected enzyme interacts with fat diglycerides, breaks down phospholipid bonds, thereby releasing unsaturated free fatty acids, which have a relatively low amount of harmful impurities.

The original strain of *Aspergillus oryzae* is known to have been used in the production of enzymes for quite some time. It was modified to produce and secrete lipase and prevent or reduce the production of unwanted secondary metabolites, which was important for its use in the food industry. Genetic modifications do not raise doubts regarding its safety. It is also known that lipases catalyze the hydrolysis of ether bonds in triacylglycerols, thereby promoting the formation of free fatty acids, diapylglycerols, and monoacylglycerols. Enzyme activity is determined using a pH status titration system and is expressed as kilolipase units/g (KLU/g), where one KLU is the amount of enzyme that releases 1 µmol of titrated butyric acid per minute under standard conditions (reaction conditions: pH=7.0 T =30°C, reaction time - at least 1.5 minutes). One LU corresponds to the Designation of International Enzyme Unit. The assay uses tributyrin as a substrate. Its breakdown produces butyric acid, which changes the pH. The reaction rate and enzyme activity are determined by measuring the volume of titrant added to the reaction system per minute to maintain a constant pH level.

Lipase is active at temperatures of up to 70°C (optimum around 40°C at pH of 6) and in the pH range from 4 to 10 (optimum around pH of 8 at 30°C). The thermal stability of the lipase was tested in the temperature range from 30°C to 90°C after 30 minutes of pre-incubation at different temperatures while maintaining pH of 6. The activity itself was measured under standard conditions. The enzyme remained active at temperatures of up to 50°C. As the temperature is increased, the enzyme loses its activity rather quickly (at 60°C the residual activity is 40%, with a 30-minute pre-incubation, maintaining pH of 6), and when the temperature is increased above 80°C, the enzyme does not retain its activity for 30 min.

Thus, regarding the use of the above-mentioned enzyme as a whole with other essential properties of the presented method, it can be stated that the temperature conditions for all stages were optimized, because due to the high activity of the enzyme, the fermentation reaction goes faster, but at a rather low temperature, thus ensuring lower energy consumption for the implementation of this method, and its lower process temperatures increase its safety, eliminate the need to use large amounts of harmful chemicals, and leave no extremely harmful by-products.

During fermentation, it is desirable to regulate the temperature to values lower than the enzyme activation temperature.

It is also desirable to mix the raw materials with water and, with mechanical stirring, heat to a temperature from 40°C to 75°C. The proportions of mixed raw materials and water - from 30% up to 55%.

The fermentation takes from 24 to 48 hours in the preferred implementation option of the present invention.

For application of this method, it is desirable to provide a stage where pre-treatment of waste intended for use as raw materials is carried out, removing suspended substances and impurities. The exact specific actions that are performed at the stage of pre-treatment of raw materials depend on the type of raw materials and the bioproduct that one wants to obtain using the presented method. Thus, for example, if particles or free water need to be removed, the raw materials are sedimented, centrifuged, and filtered during the pre-treatment stage. Emulsifiers and soap are removed by rinsing with water, mineral acids are neutralized by diluting with aqueous solution of NaOH. Obviously, these examples are illustrative and not exhaustive.

It is preferable to isolate at least one bioproduct by gravity sedimentation of the mixture after the fermentation has taken place in order to separate the phases.

Also, when using this method, it is desirable to allow for a stage when, after the fermentation process is over, the aqueous enzyme solution is separated from the unfermented waste intended for use as raw material, in order to reuse it in subsequent cycles of implementation of this method. This would allow economical use of reagents and ensure lower operating costs when implementing this method.

The preferred version of the application of the presented invention, the processing by-products from oil and fat industry are used as fats, oils and their fractions contained in waste intended for use as raw materials.

Preferably that the bioproduct obtained from vegetable oils and free fatty acids would not be limited to the examples given. Thus, the presented method can be used for the production of protein and amino acid concentrate biofuel, etc.

Implementation process of the method allowing to obtain at least one bioproduct from waste intended for use as raw materials:
By-products of oil and fat industry processing are used for the implementation of the presented method, namely waste intended for use as raw materials containing at least one of the following substances: fats, oils, and their fractions. For example, hydration precipitate, which are formed in oil factories as a secondary by-product after the chemical purification of various types of vegetable oils by hydrolysis, can be used as such raw material. It can also be precipitate, which is a by-product of the production of unrefined sunflower oil, and which contains fats and proteins, or flotation waste, i.e. a mixture of water, small, solid proteins and fats of animal origin, which is formed during the treatment of industrial effluents.

Further, if necessary, pre-treatment of the chosen waste intended for use as raw materials is carried out, removing suspended substances and impurities. The fermentation process requires careful control of certain parameters related to the quality of the raw materials. The most common examples of pre-treatment of waste intended for use as raw materials are sedimentation at 60°C to separate free water and heavy solids by decantation or centrifugation, filtration (filtration degree is 10 microns) to remove residual solids, rinsing with 5-10% of water at 80-90°C to remove emulsifiers and water-soluble contaminants, centrifugation to remove wash water and emulsifiers, rapid drying to extract water below 5000 ppm, neutralization of mineral acids with aqueous solution of NaOH.

After appropriate pre-treatment, the waste intended for use as raw materials is placed in the reactor of the system, where the main processes of the presented method take place. Water is also added there, the proportion of which is from 30 % to 55 % of the raw material mass, after which the resulting mixture is heated with mechanical stirring. At the same time, the pressure vessel of the respective system is filled with enzyme, a liquid lipase product obtained from the genetically modified microorganism *Aspergillus oryzae.* The amount of enzyme is determined for each type of raw material separately, but preferably it should be at least 3 kg per ton of raw material. After reaching the appropriate temperature in the reactor - not lower than 40°C, and preferably 42°C - the mentioned enzyme is dripped from the pressure vessel.

Also, depending on the final bioproduct to be obtained, other reagents may be added. After adding all the reagents and reaching a temperature of 48°C-52°C (if the waste intended for use as raw material is sediment or flotation waste), or 55°C (if the waste intended for use as raw material is sediment), the contents of the reactor are stirred for 24 hours, then the stirring mechanism is turned off. At the same time during fermentation, it is ensured that the pH of the contents of the reactor would be 6-7. During the reaction, the enzyme interacts with fat diglycerides, breaking phospholipid bonds, thereby releasing unsaturated free fatty acids. At the end of the fermentation reaction, the contents of the reactor are sedimented directly inside the reactor or brought to a special container and then sedimented in this container in order to separate the phases, for example, free fatty acids - defatted sediment - returned water, oil - hydration sediment, etc. The amount of isolated free fatty acids, depending on the raw material, can vary from 15% to 90%. Meanwhile, in the obtained bioproduct, i.e. in the sample of free fatty acids obtained, after conducting laboratory tests, it was found that it contains such harmful impurities as sulfur and phosphorus, with a concentration of 2 ppm and 17 ppm, respectively, when the limit values of the concentration of these elements according to the current technical standards (ISO 7580-91 , ISO 16958-2018) are up to 40 ppm and up to 100 ppm, respectively.

The resulting target bioproduct is filtered, centrifuged, or separated from the rest of the precipitated components by other acceptable means. The resulting bioproduct can then be purified in order to remove residual enzymes and other impurities before being used for further processing or production.

Thus, a method that allows obtaining at least one bioproduct from waste intended for use as raw materials was developed, which, after optimally choosing the conditions of its application, would ensure a technical result that would allow optimizing the temperature conditions during the implementation of the method steps, reducing the fermentation time, reducing energy costs, and obtaining a bioproduct with relatively low amount of harmful impurities.

## Claims

1. A method for obtaining at least one bioproduct from waste intended for use as raw materials, provides for the following stages: the waste intended for use as a raw material, which contains at least one of the following substances: fats, oils or their fractions, is supplied; the raw material is mixed with water and heated to a temperature not lower than 40°C by mechanical stirring; enzyme is added; fermentation takes place under stirring and adjusting the temperature of the mixture, and at least one bioproduct is obtained; at least one bioproduct is separated. The liquid lipase product obtained from the genetically modified microorganism *Aspergillus oryzae* is used as an enzyme.

2. The method according to claim 1, **characterised in that** the liquid lipase product is a hydrolase of carboxylic acid ester obtained by immersing the genetically modified microorganism *Aspergillus oryzae* in water for fermentation.

3. The method according to claim 1, **characterised in that** during fermentation the temperature is regulated to values lower than the enzyme activation temperature.

4. The method according to claim 1, **characterised in that** the raw material is heated to a temperature from 40°C to 75°C during mechanical stirring.

5. The method according to claim 1, **characterised in that** the fermentation lasts from 24 to 48 hours.

6. The method according to claim 1, **characterised in that** it provides a stage in which the raw materials are pre-treated in order to remove suspended waste intended for use as raw materials and impurities.

7. The method according to claim 1, **characterised in that** at least one bioproduct is separated by gravity sedimentation of the mixture after the fermentation has been carried out in order to separate the phases.

8. The method according to claim 1, **characterised in that** a stage is provided for after the fermentation process is over, the aqueous enzyme solution is separated from the unfermented waste intended for use as raw materials in order to reuse it.

9. The method according to claim 1, **characterised in that** the processing by-products from oil and fat industry are used as fats, oils and their fractions contained in waste intended for use as raw materials.

10. The method according to claim 1, **characterised in that** a bioproduct is obtained - at least one of vegetable oils and free fatty acids.
